# EUROPEAN PATENT APPLICATION

(11) **EP 3 498 151 A1**
(43) Date of publication of application: **19.06.2019**
(21) Application number: 17207357.9
(22) Date of filing: 14.12.2017
(51) Int. Cl.: A61B 5/00

(54) **METHOD AND APPARATUS FOR DETERMINING ONSET OF SLEEP**

(71) Applicant: Koninklijke Philips N.V., 5656 AE Eindhoven (NL)
(72) Inventor: VAN EE, Raymond, 5656 AE Eindhoven (NL); JOHNSON, Mark Thomas, 5656 AE Eindhoven (NL); TEN KATE, Warner Rudolph Theophile, 5656 AE Eindhoven (NL); VAN DE WOUW, Doortje, 5656 AE Eindhoven (NL); AARTS, Ronaldus Maria, 5656 AE Eindhoven (NL)
(74) Representative: de Haan, Poul Erik

(57) **Abstract**

According to an aspect, there is provided an apparatus for monitoring a subject, the apparatus comprising a processing unit that is configured to obtain measurements of of measurements to identify one or more periods of time in which the subject has fallen asleep, wherein the one or more periods of time in which the subject has fallen asleep correspond to periods of time in which the neck muscle is relaxed.

## Description

### FIELD OF THE INVENTION

The invention relates to a method and apparatus for monitoring a subject, and in particular relates to monitoring a subject to determine when the subject has fallen asleep.

### BACKGROUND TO THE INVENTION

Variability and fragmentation in daily behaviours has been identified as a risk indicator for healthy ageing. One example of such a behaviour is variability in sleeping during the day, which is often referred to as dozing or napping (i.e. to sleep lightly and intermittently). Dozing typically occurs while a person is sitting on a chair or sofa. It has been found that variability and fragmentation in dozing and napping is associated with shorter life expectations.

In view of the association between variability and fragmentation of sleeping during the day with life expectancy, it is desirable for subjects, care providers and/or family members to monitor whether, when and/or for how long a subject is sleeping during the day.

### SUMMARY OF THE INVENTION

It has been found that a particularly characteristic feature of sleeping during the day (particularly when the subject is sitting on a chair or sofa) is that during sleeping (dozing) the muscles in the neck of the subject relax almost completely. Depending on the position of the subject's head when they fall asleep, this relaxation can lead to a sudden nodding of the head (i.e. a flexion of the neck in which the chin moves towards the chest). This relaxation of the neck muscles does not occur when the subject is sitting still but awake, for example when reading a book or watching television, as in this case the muscles are continuously being activated to maintain the subject in an upright posture. Aspects of this disclosure make use of this characteristic of sleeping (dozing) to monitor when a subject has fallen asleep.

Thus, according to a first aspect, there is provided an apparatus for monitoring a subject, the apparatus comprising a processing unit that is configured to obtain measurements of a level of relaxation of a neck muscle of the subject over time; and process the measurements to identify one or more periods of time in which the subject has fallen asleep, wherein the one or more periods of time in which the subject has fallen asleep correspond to periods of time in which the neck muscle is relaxed. Therefore it is possible to identify periods of time in which the subject has fallen asleep, particularly periods of time where the subject is dozing or snoozing in a chair, or otherwise in a sitting position.

In some embodiments, the processing unit is configured to obtain the measurements of the level of relaxation of the neck muscle from a memory unit. In this way the measurements are not required to be processed in real time and can be processed as and when it is desired to identify periods of time in which the subject has fallen asleep.

In alternative embodiments, the processing unit is configured to obtain the measurements of the level of relaxation of the neck muscle by processing a signal from one or more sensors. In this way the periods of time in which the subject has fallen asleep can be identified in real-time or near real-time.

In some embodiments, the one or more sensors comprise one or more of an electromyography (EMG) sensor, an accelerometer, a gyroscope, a strain gauge, a sensor that measures the indentation of a probe into the neck muscle, a sensor that measures vibration of the muscle from an external stimulus and a camera.

In some embodiments, the apparatus comprises the one or more sensors.

In some embodiments, the one or more sensors are worn or carried by the subject.

In some embodiments, the apparatus is configured to be worn at or around the neck of the subject. This enables the apparatus to be worn by the subject relatively unobtrusively.

In some embodiments, the apparatus comprises a band, cord or strap that is to be worn around the neck of the subject, and wherein the one or more sensors are arranged in or on the band, cord or strap such that, when worn by the subject (in use by the subject), the one or more sensors are positioned adjacent to the neck muscle of the subject. In this way the sensors can be included in the apparatus relatively unobtrusively and directly measure the relaxation level of the muscle.

In some embodiments, the processing unit is further configured to process the measurements of the level of relaxation of the neck muscle of the subject and measurements of one or more further characteristics of the subject to identify one or more periods of time in which the subject has fallen asleep. The use of one or more further characteristics in combination with the muscle relaxation level can improve the reliability of the sleep period identification.

In some embodiments, the measurements of one or more further characteristics of the subject comprise one or more of a movement or activity level of the subject, a physiological characteristic of the subject, heart rate, heart rate variability, breathing rate, and skin conductivity.

In some embodiments, the processing unit is configured to obtain the measurements of the one or more further characteristics by (i) processing a signal from one or more sensors to determine the measurements, or (ii) obtaining the measurements from a memory unit.

In some embodiments, the processing unit is further configured to determine a measure of the regularity or variability of a plurality of identified periods of time in which the subject has fallen asleep. This analysis provides a way to monitor the subject's sleeping pattern over time.

In some embodiments, the processing unit is further configured to compare the determined measure of the regularity or variability to a threshold value; and provide an indication, notification or message to a user if the measure of the regularity or variability exceeds the threshold value.

According to a second aspect, there is provided a method of monitoring a subject, the method in a processing unit comprising obtaining measurements of a level of relaxation of a neck muscle of the subject over time; and processing the measurements to identify one or more periods of time in which the subject has fallen asleep, wherein the one or more periods of time in which the subject has fallen asleep correspond to periods of time in which the neck muscle is relaxed. Therefore it is possible to identify periods of time in which the subject has fallen asleep, particularly periods of time where the subject is dozing or snoozing in a chair, or otherwise in a sitting position.

In some embodiments, the step of obtaining comprises obtaining the measurements of the level of relaxation of the neck muscle from a memory unit. In this way the measurements are not required to be processed in real time and can be processed as and when it is desired to identify periods of time in which the subject has fallen asleep.

In alternative embodiments, the step of obtaining comprises obtaining the measurements of the level of relaxation of the neck muscle by processing a signal from one or more sensors. In this way the periods of time in which the subject has fallen asleep can be identified in real-time or near real-time.

In some embodiments, the one or more sensors comprise one or more of an electromyography (EMG) sensor, an accelerometer, a gyroscope, a strain gauge, a sensor that measures the indentation of a probe into the neck muscle, a sensor that measures vibration of the muscle from an external stimulus and a camera.

In some embodiments, the one or more sensors are worn or carried by the subject.

In some embodiments, the one or more sensors are arranged in a band, cord or strap of an apparatus that is to be worn around the neck of the subject, and the one or more sensors are arranged in or on the band, cord or strap such that the one or more sensors are positioned adjacent to the neck muscle of the subject. In this way the sensors can be included in the apparatus relatively unobtrusively and directly measure the relaxation level of the muscle.

In some embodiments, the step of processing comprises processing the measurements of the level of relaxation of the neck muscle of the subject and measurements of one or more further characteristics of the subject to identify one or more periods of time in which the subject has fallen asleep. The use of one or more further characteristics in combination with the muscle relaxation level can improve the reliability of the sleep period identification.

In some embodiments, the measurements of one or more further characteristics of the subject comprise one or more of a movement or activity level of the subject, a physiological characteristic of the subject, heart rate, heart rate variability, breathing rate, and skin conductivity.

In some embodiments, the method further comprises the step of obtaining the measurements of the one or more further characteristics by (i) processing a signal from one or more sensors to determine the measurements, or (ii) obtaining the measurements from a memory unit.

In some embodiments, the method further comprises the step of determining a measure of the regularity or variability of a plurality of identified periods of time in which the subject has fallen asleep. This analysis provides a way to monitor the subject's sleeping pattern over time.

In some embodiments, the method further comprises the steps of comparing the determined measure of the regularity or variability to a threshold value; and providing an indication, notification or message to a user if the measure of the regularity or variability exceeds the threshold value.

According to another aspect of the invention, it is provided a computer program product comprising a computer readable medium having computer readable code embodied therein, the computer readable code being configured such that, on execution by a suitable computer or processor, the computer or processor is caused to perform the method according to the second aspect or any of the embodiments described above.

These and other aspects of the invention will be apparent from and elucidated with reference to the embodiment(s) described hereinafter.

### BRIEF DESCRIPTION OF THE DRAWINGS

Exemplary embodiments of the invention will now be described, by way of example only, with reference to the following drawings, in which:
Figure 1 is a block diagram of an apparatus according to an embodiment and a sensor unit;
Figure 2 illustrates an exemplary arrangement of a sensor on a subject according to an embodiment;
Figure 3 illustrates a sensor that measures the relaxation of a muscle using a probe;
Figure 4 illustrates a sensor that measures the relaxation of a muscle using a stimulus; and

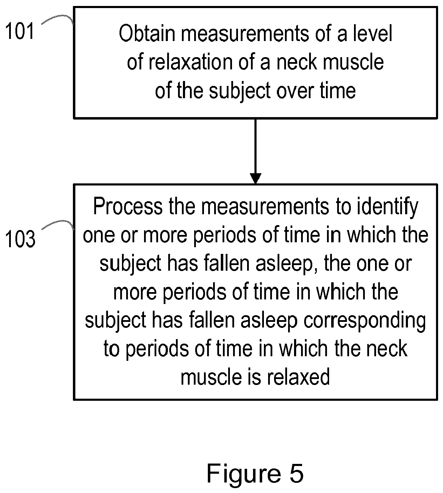

Figure 5 is a flow chart illustrating a method according to an embodiment.

### DETAILED DESCRIPTION OF EMBODIMENTS

As noted above, it has been found that a particularly characteristic feature of sleeping that can be used to identify when the subject is sleeping during the day is that the muscles in the neck of the subject relax almost completely during sleep. The invention therefore provides that measurements of the level of relaxation of a neck muscle or neck muscles of a subject are evaluated to identify periods of time in which the subject has fallen asleep. The terms 'asleep', 'dozing', 'snoozing' and 'napping' are used interchangeably in this disclosure to refer to a subject that is sleeping.

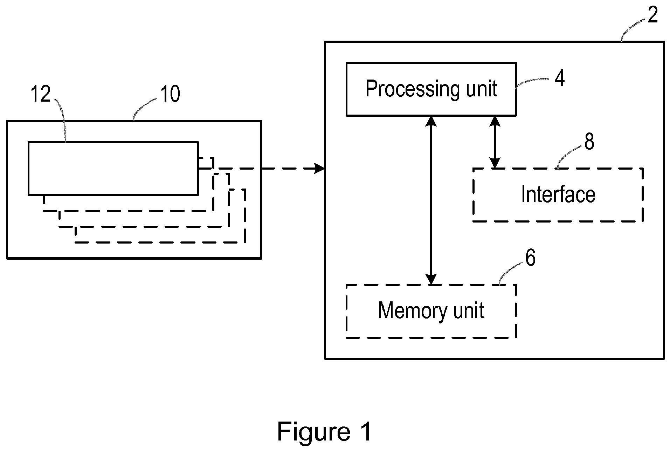

Figure 1 shows an apparatus 2 for monitoring a subject according to an embodiment, and specifically for monitoring a subject to identify periods of time in which the subject has fallen asleep. The apparatus 2 comprises a processing unit 4 that controls the operation of the apparatus 2 and generally implements the method according to the invention. The processing unit 4 can be implemented in numerous ways, with software and/or hardware, to perform the various functions described below. The processing unit 4 may comprise one or more microprocessors or digital signal processor (DSPs) that may be programmed using software or computer program code to perform the required functions and/or to control components of the processing unit 4 to effect the required functions. The processing unit 4 may be implemented as a combination of dedicated hardware to perform some functions (e.g. amplifiers, preamplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

In various implementations, the processing unit 4 may be associated with or comprise one or more memory units 6 such as volatile and non-volatile computer memory such as RAM, PROM, EPROM, and EEPROM. The processing unit 4 or associated memory unit 6 can also be used for storing program code that can be executed by a processor in the processing unit 4 to perform the methods described herein. In some embodiments, the memory unit 6 can store information generated during execution of the methods, information for use during execution of the methods (e.g. measurements from one or more sensors), the output(s) of the method (e.g. an indication of periods of time that the subject was asleep).

The apparatus 2 also comprises an interface 8 for outputting the indication of periods of time that the subject was asleep. The interface 8 may be a user interface for outputting the indication determined by the processing unit 4 to a user of the apparatus 2. Depending on the way in which the apparatus 2 is implemented, the user of the apparatus 2 may be the subject themselves (i.e. the subject being monitored), or a clinician or care provider. The user interface 8 can therefore be or comprise a display screen or one or more other elements (e.g. lights or LEDs) for providing a visual output, a loudspeaker for providing an audible output, a vibrating element for providing a tactile output, or any combination thereof. The user interface 8 may also enable the user to interact with the apparatus 2, for example to activate or deactivate the apparatus 2 and/or to control one or more settings or operations of the apparatus 2. As such the interface 8 can comprise any one or more of a touch screen, button(s), switch(es), keypad, keyboard, mouse, trackpad, stylus, etc. As an alternative or in addition to a user interface, the interface 8 may be an interface for allowing the apparatus 2 to exchange information with another electronic device (e.g. a computer or server). In that case the interface 8 may enable the apparatus 2 to establish a wired or wireless connection with the other electronic device using any suitable communication protocol or communication technology (for example USB, Ethernet, Zigbee, Bluetooth, WiFi, a wide-area network technology, a cellular telecommunication technology such as Global System for Mobile communications (GSM), Universal Mobile Telecommunications Service (UMTS) or Long Term Evolution (LTE), etc.).

In order for the processing unit 4 to identify periods of time in which the subject has fallen asleep, measurements of a level of relaxation of a neck muscle of the subject are required. Figure 1 shows a sensor unit 10 that comprises one or more sensors 12 for providing measurements of the level of relaxation of the neck muscle of the subject over time.

In some embodiments (e.g. as shown in Figure 1), the sensor unit 10 is separate from the apparatus 2, and the measurements are provided from the sensor unit 10 to the apparatus 2, for example via the interface 8. The measurements can be provided to the apparatus 2 in real-time, or the sensor unit 10 can periodically or intermittently provide the measurements to the apparatus 2. In some embodiments, the measurements from the sensor unit 10 can be stored in a memory unit or database, and the apparatus 2 can obtain or retrieve the measurements from the memory unit or database when it is desired to determine a sleep status or identify periods of time in which the subject was asleep.

The sensor unit 10 may include a single sensor 12, multiple sensors 12 of the same type or multiple sensors 12 of different types. The sensor unit 10 can include one or more of an electromyography (EMG) sensor, an accelerometer, a gyroscope, a strain gauge, a sensor that measures the indentation of a probe into the neck muscle, a sensor that measures vibration of the muscle from an external stimulus and a camera. Those skilled in the art will be aware of other types of sensors that can be used. Further explanation of the way in which each of these sensors can be used to measure the level of relaxation of the neck muscle of the subject over time is provided below.

As noted above, in some embodiments all of the components, e.g. the processing unit 4, memory unit 6, interface 8, sensor unit 10, sensor(s) 12, can be contained within the apparatus 2. For example the apparatus 2 could be in the form of a smartphone, tablet computer, pendant, necklace, bracelet, smart watch, etc. that can be carried or worn by the subject. In this case the sensor(s) 12 can be one or more of the electromyography (EMG) sensor, accelerometer, gyroscope, strain gauge, the sensor that measures the indentation of a probe into the neck muscle and the sensor that measures vibration of the muscle from an external stimulus. Alternatively, the apparatus 2 could be in a form that is suitable to be placed in or fixed to the environment of the subject (for example attached to a wall in a room in which the subject typically dozes, such as a living room). In this case the sensor(s) 12 can be a camera.

Also as noted above, in some embodiments the sensor unit 10 is separate from the apparatus 2. In these embodiments, the apparatus 2 may be local to the sensor unit 10, for example in wired or wireless communication with the sensor unit 10, or it may be remote from the sensor unit 10. For example the apparatus 2 could be, or be part of, a computer, server, laptop, tablet computer, etc. In some embodiments, the functions of the apparatus 2 could be implemented by a distributed arrangement of computers/servers. In embodiments where the sensor unit 10 is separate from the apparatus 2, the sensor unit 10 can be in the form of a wearable or portable device, such as a smartphone, tablet computer, pendant, necklace, bracelet, smart watch, etc. In this case the sensor(s) 12 can be one or more of the electromyography (EMG) sensor, accelerometer, gyroscope, strain gauge, the sensor that measures the indentation of a probe into the neck muscle and the sensor that measures vibration of the muscle from an external stimulus. Alternatively, the sensor unit 10 could be in a form that is suitable to be placed in or fixed to the environment of the subject (for example attached to a wall in a room in which the subject typically dozes, such as a living room). In this case the sensor(s) 12 can be a camera.

As described in more detail below, various ones of the types of sensors 12 that can be used to measure the level of relaxation of the neck muscle of the subject are required to be located near to the neck of the subject, at the neck of the subject or on the neck of the subject. Therefore, in some preferred embodiments, the sensor unit 10 is in the form of, or is part of, a pendant or necklace that can be worn around the neck of the subject. In these embodiments, the sensor unit 10 can be the part of a pendant that hangs below the head of the subject, or the sensor unit 10 can be part of the band, cord or strap of a pendant or necklace that is worn around the neck of the subject. In this way the one or more sensor(s) 10 can be positioned adjacent to the neck muscle of the subject, for example touching the skin of the subject's neck.

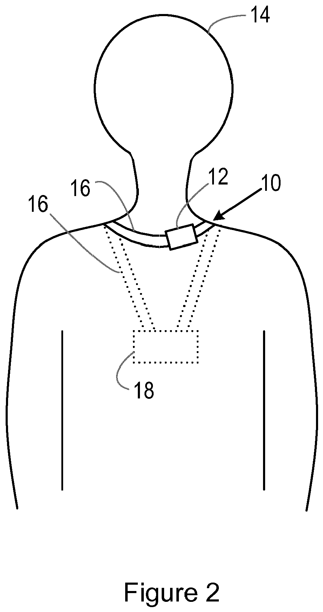

Figure 2 illustrates an exemplary arrangement of a sensor 12 on a subject 14 according to an embodiment. Figure 2 shows the back of the subject 14. In this embodiment the sensor unit 10 is in the form of a pendant that has a band or cord 16 that is placed around the neck of the subject 14. The pendant has a portion 18 that hangs on the front of the subject 14. In some embodiments, the portion 18 is decorative, i.e. it does not have a specific technical function, but in other embodiments the portion 18 can include circuitry associated with the sensor 12 or sensor unit 10, or other components that perform a technical function. For example the portion 18 could comprise components for a personal help button (PHB) and/or components that monitor the general movements of the subject 14, for example to detect if the subject 14 suffers a fall. In some embodiments, the portion 18 can be or comprise the apparatus 2 for processing the measurements from the sensor 12.

In Figure 2 the sensor 12 is arranged on the band 16 so that it contacts the skin on the back of the neck of the subject 14. The sensor 12 may use an adhesive to hold the sensor 12 in place on the skin of the neck, or the sensor 12 may be pushed or held onto the skin by the arrangement of the band 16 (for example if the band 16 is configured to apply the sensor 12 to the skin with some pressure), or the weight of the portion 18 can help to apply the sensor 12 to the skin with a required pressure. In some embodiments, the sensor 12 is part of or housed in the band or cord 16, preferably on the inside of the band or cord (so that the sensor 12 faces the skin of the subject 14 when the pendant is being worn).

Of the different types of sensors 12 that can be used in the sensor unit 10, some of the sensors 12 can directly measure the level of relaxation of the muscle(s). For example, an EMG sensor, a strain gauge, a sensor that measures the indentation of a probe into the neck muscle and a sensor that measures vibration of the muscle from an external stimulus can all directly measure the level of relaxation of the muscle(s). Other sensors 12, such as an accelerometer, a gyroscope, and a camera, can indirectly measure the level of relaxation of the muscle(s) by measuring or observing the sudden nodding motion of the head of the subject. In particular, the head of the subject often nods forwards uncontrollably when the muscles in the neck relax due to the subject dozing off, and the measurements or observations of these nodding movements (or absence of such movements) by an accelerometer, gyroscope or camera can be used to infer the level of relaxation of the muscles in the neck.

In the case of an EMG sensor, the sensor 12 can comprise one or more electrodes that are arranged on the band 16 so that they contact the skin on the back of the neck of the subject 14. These electrodes are used to measure electrical signals from the muscles in the neck of the subject 14. The electrodes may be any suitable type of electrodes, and may use an adhesive to hold them in place on the skin of the neck, or they may be pushed or held onto the skin by the arrangement of the band 16 (for example if the band 16 is configured to apply the electrodes 20 to the skin with some pressure), or the weight of the portion 18 can help to apply the electrodes 20 on to the skin with a required pressure. In general, the more active (i.e. tense) the muscle, the higher the EMG signal that is measured by the electrodes 20. Thus, when the muscle is relaxed, the measured EMG signal is much lower than when the muscle is active. An EMG sensor is useful as it typically has a small size and provides a readily detectable or processable signal. In particular embodiments, the EMG sensor is a GaN-based transistor with an exposed transistor gate, but other known types of EMG sensors could be used instead.

In embodiments where the sensor 12 is an accelerometer, the accelerometer could be positioned so that it is on the back of the neck of the subject 14 (e.g. as shown in Figure 2) so that it can measure the accelerations and orientation change (i.e. by observing a change in the direction of acceleration due to gravity in the reference frame of the accelerometer) associated with the uncontrolled nodding of the head of the subject 14 when the muscles of the neck relax. During such a nod, the angle of the head can rotate around 70°-90° from the vertical, and the speed of the nod will increase. Alternatively, an accelerometer can be located in the portion 18 and can measure the acceleration/movement of the portion 18 due to the nodding of the head of the subject 14.

In embodiments where the sensor 12 is a gyroscope, the gyroscope could be positioned so that it is on the back of the neck of the subject 14 (e.g. as shown in Figure 2) so that it can measure the orientation change associated with the uncontrolled nodding of the head of the subject 14 when the muscles of the neck relax. Alternatively, a gyroscope can be located in another head-mounted device, such as a pair of glasses or a hearing aid.

In embodiments where the sensor 12 is a strain gauge, the strain gauge could be positioned so that it is on the front, side or back of the neck of the subject 14 (e.g. as shown in Figure 2), for example located in the band or cord 16. The strain gauge measures the elasticity of the neck muscles. As the tension in the muscles in the neck changes, the strain in the band or cord 16 will also change, and this can be measured by the strain gauge. As the muscles in the neck relax, the strain in the band or cord 16 will decrease. On the other hand, when the muscles in the neck are tensed (e.g. when supporting the head in an upright position) the strain will be higher.

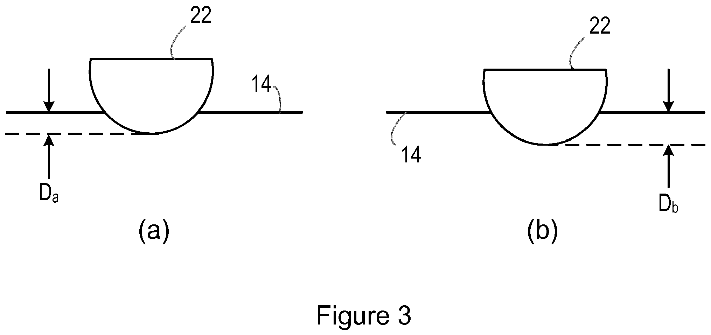

Figure 3 illustrates how a sensor 12 that measures the indentation of a probe into the neck muscle can be used to measure the level of relaxation of the muscle. As shown in Figure 3(a) a probe 22 is placed in contact with the skin of the subject 14. A force is applied to the probe 22 so that it presses on to the skin of the subject 14. The applied force is preferably constant so that the measure of the relaxation of the muscle can be determined based on the amount of indentation. Due to the elasticity of the skin and underlying muscle, the probe 22 creates an indentation in the skin beneath the probe 22, that is the probe 22 presses the skin and muscle into the body of the subject. In Figure 3(a) the underlying muscle is tense (for example when the head of the subject is being supported), and the probe 22 has formed an indentation that has a depth denoted Dₐ. In Figure 3(b) the underlying muscle is relaxed (or more relaxed than in Figure 3(a)), and the probe 22 has formed a deeper indentation that has a depth denoted D_{b} (where D_{b} > Dₐ). Thus, the sensor 22 can measure the amount of indentation, and the measurements of the indentation can be used to indicate the level of relaxation of the muscle(s). In addition, or alternatively, a ratio can be determined between the force F applied to the probe 22 and amount of indentation D. This ratio is referred to as an elasticity coefficient k (and is derived from Hooke's law where the exercised force F = elasticity coefficient k * amount of indentation D). In these cases, the amount of the applied force can be variable, either or both in terms of the magnitude of the force and frequency with which the force is applied (including static, i.e. 0 Hz). The probe 22 can be a hemispherical protrusion, or have any other suitable shape or configuration. One approach to measure the indentation D is to use a stretch sensor that covers an exciter. It could also connect to an end point of a cantilever (the exciter can be embodied as a cantilever that bends with excitation, e.g. using Electroactive Polymers, EAPs). In case of a probe 22 that is pushed out by a coil, the stretch sensor could connect to the upper part of the probe 22 (the lower part of the probe 22 being the side that pushes into the skin).

In case the exciter is a ferromagnetic element inside a coil, i.e. it pushed out by the driving current, the self-inductance could be measured (e.g. as the ratio of driving force and current over the coil). Alternatively, the ferromagnetic element could move inside a second coil when pushed out by the first coil, in this way forming a transformer with the part of the coil it is moving into. Induced output voltage at the second coil would indicate the displacement D.

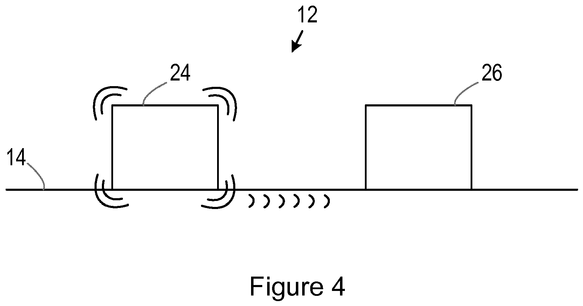

In embodiments where the sensor 12 measures vibration of the muscle from an external stimulus, the sensor 12 could be positioned so that it is on the front, side or back of the neck of the subject 14 (e.g. as shown in Figure 2), for example located in the band or cord 16. As shown in Figure 4, the sensor 12 can comprise a stimulus component 24 for applying a stimulus, such as an impulse or a vibration, to the part of the body having the muscle of interest (e.g. to the skin at the neck of the subject 14), and a sensor component 26 for measuring the response of the muscle to the stimulus. The sensor component 26 measures the response at a different position on the subject 14 to the position where the stimulus component 24 is used (although the difference in position will be small, e.g. a few mm). In some embodiments, the sensor component 26 is an accelerometer. In other embodiments, the sensor component 26 can be a ferromagnetic element inside a coil, with the ferromagnetic element being extended from the coil through a current being applied to the coil and in contact with the skin. The impedance of the coil could be measured, and this is related to a ratio of the muscle tension to the current. The level of tension/relaxation of the muscles determines the degree of damping of the stimulus (e.g. the amplitude and/or peak frequency of the stimulus) as measured by the sensor component 26. In particular, the more relaxed the muscles are, the lower the amplitude measured by the sensor component 26, and the peak frequency tends to zero. In some embodiments, the stimulus component 24 can be configured to apply a vibration that satisfies a resonance condition when the (neck) muscles are tense (although it should be noted that the frequency of vibration does not need to be a resonant frequency of the tensed muscle), which means that the decrease in vibration amplitude that occurs when the muscle relaxes will be larger, and thus easier to measure. Thus, with the sensor 12 in Figure 4, the level of relaxation of a neck muscle of the subject 14 can be determined from measurements of the stimulus, and in particular measurements of the amplitude, peak frequency and/or other measure of the damping of the stimulus by the muscle.

In embodiments where the sensor 12 is a camera, the camera can be located in the environment of the subject 14, for example in a room of their home or care environment, so that it can observe the subject 14. Multiple cameras can be located in a particular room, and/or cameras can be located in different rooms. Sequences of images obtained by the camera can be processed using image processing techniques to detect the subject and to determine the head movements of the subject. As noted above, when the muscles of the neck relax, the head can nod forward uncontrollably. By analysing the head movements of the subject, it is possible to identify when this uncontrolled nod has occurred. Characteristics of the head movements of the subject 14 that can be extracted and analysed include the angle of the head/neck with respect to the body, the speed of motion and the acceleration of the motion. It may also be possible for the eye movements or eyelid position (e.g. opened/closed) to be extracted from the images, which can also be used to determine or confirm whether the subject 14 has fallen asleep.

It will be appreciated that the apparatus 2 may comprise additional components to those shown in Figure 1. For example the apparatus 2 may comprise a power source, such as a battery, or a power interface component, such as plug, for connecting to the apparatus 2 to a mains power supply. In embodiments where the sensor unit 10 is separate from the apparatus 2, the sensor unit 10 may also comprise a power source, such as a battery, or a power interface component, such as plug, for connecting to the sensor unit 10 to a mains power supply.

In certain embodiments, the sensor unit 10 can comprise a sensor 12 for measuring the level of relaxation of the neck muscle and one or more additional sensors can be carried or worn by the subject 14 (e.g. at another part of the body) or provided in the environment of the subject 14 that can provide further measurements of the subject 14 to the apparatus 2, and that can be processed with the muscle relaxation measurements to determine if the subject has fallen asleep. These additional sensors may be separate from the sensor unit 12 and apparatus 2, and for example may be part of a smart phone or smart watch. In addition or alternatively, the additional sensor can include a passive infra-red (PIR) sensor that can provide a signal indicating whether motion is detected by the PIR sensor. This motion information can be used in a similar way to motion information from a camera or an accelerometer.

Figure 4 illustrates a method of monitoring a subject according to an aspect of the invention. The method in Figure 4 can be performed by the apparatus 2, and specifically by the processing unit 4, optionally as a result of executing program code that is stored in the memory unit 6.

In a first step, step 101, measurements of the level of relaxation of a neck muscle of the subject 14 are obtained. This step can comprise receiving measurements from the sensor 12 or the sensor unit 10, for example in real-time or near real-time, or retrieving a set of previously-stored measurements from memory unit 6. The former case makes it possible for the apparatus 2 to determine whether the subject 14 is asleep/nodded off in real-time or near-real-time. However, generally the daily sleep routine may only need to be evaluated once per day, once per week, etc., and so measurements can be stored for processing at a later stage.

The measurements of the level of relaxation of the neck muscle of the subject 14 obtained in step 101 may be raw sensor measurements, e.g. a signal or set of samples as output by the sensor 12, or the measurements may be pre-processed in some way, for example filtering to remove noise, etc. In either case, step 101 may comprise performing some processing on the sensor signal from the sensor(s) to determine the measurements of the level of relaxation of the neck muscle of the subject 14. For example, step 101 can comprise comparing an EMG signal amplitude, a probe indentation depth, a measured strain, or a peak frequency/amplitude (as appropriate for the type of sensor 12) to one or more threshold values to determine a measurement of a level of relaxation, or step 101 can comprise determining an amount of orientation change or rotation of the head of the subject 14 from acceleration or gyroscope measurements and comparing the determined amount of orientation change/rotation to one or more thresholds to determine a measurement of the level of relaxation.

Next, in step 103, the measurements are processed to identify one or more periods of time in which the subject 14 has fallen asleep. In particular those periods of time in which the measured level of relaxation indicates that the neck muscle is relaxed are considered as one or more periods of time in which the subject 14 has fallen asleep.

Thus, for example, in the case of EMG measurements, step 103 can comprise identifying a period of time in which the subject 14 has fallen asleep as a period of time in which the EMG signal is below a threshold value that indicates that the neck muscle is relaxed. The relaxation of the neck muscle in a dozing/nodding off may occur quite quickly, for example the muscle may relax from a tense state to a relaxed state in a short time period, such as 2 seconds or less, or 1 second or less. In that case, step 103 can comprise identifying a period of time in which the subject 14 has fallen asleep as a period of time in which the EMG signal is below a threshold value, and where the EMG signal changed from indicating a tensed muscle state to a relaxed muscle state in less than a predetermined time period, for example in less than 2 seconds or for example in less than 1 second.

In the embodiments where the sensor unit 10 comprises multiple sensors 12, the measurements of the level of muscle relaxation from the different sensors 12 can each be processed in step 103 to determine the level of relaxation of the neck muscle of the subject 14, and the outcomes of this processing can be combined in step 103, for example using a classifier, to determine the period or periods of time in which the subject 14 is or has been asleep.

In further embodiments, the measurements of the level of relaxation of the neck muscle of the subject 14 can be combined with measurements of further characteristics of the subject 14 to improve the reliability of the identification of the periods of time in which the subject 14 is asleep. The further characteristics of the subject 14 can include characteristics relating to the movements or physical activity level, or physiological characteristics that can be indicative of sleeping or wakeful states, such as heart rate, heart rate variability, breathing rate, skin conductivity, etc. (where reductions in heart rate, breathing rate and heart rate variability can indicate sleeping). In some cases, i.e. depending on the type of sensor 12 in the sensor unit 10, the further characteristics of the subject 14 can be determined by processing the measurements or sensor signals obtained in step 101. Alternatively, there may be one or more further sensors in addition to sensor 12, and the method can include obtaining further measurements of the subject 14, and processing those further measurements to determine one or more further characteristics of the subject. In either case, step 103 can comprise processing those further measurements of the subject 14 along with the measurements of the level of relaxation of the subject 14 to identify periods of time in which the subject 14 has fallen asleep. In some cases the measurements can be input to a classifier that can identify periods of time in which the subject 14 is asleep.

In an example, measurements of the activity and/or motion of the subject 14 around any period of time in which the neck muscle of the subject 14 has relaxed can be used to identify whether the 'relaxed neck' period is a period of sleeping. If the measurements of the activity and/or motion indicate that the relaxed neck period was immediately preceded and/or followed by a period in which the subject 14 was relatively still/motionless, then this increases the likelihood that the subject 14 was asleep during the period that the neck muscle was relaxed. A relatively still/motionless period can be identified as a period where the magnitude of the movement is low (e.g. below a threshold value), and/or where the variance in the movement level is low (e.g. below a threshold value). It will be appreciated that the activity and/or motion measurements can be derived from an accelerometer, and the accelerometer may be the same sensor as that used to measure the level of relaxation of the neck muscle(s), or it may be a different sensor, for example located in a smart phone or smart watch that is carried or worn by the subject 14.

If one or more periods of time are identified in which the subject 14 was asleep, the method may comprise determining and/or storing information on each sleep period, such as the start time of the period, the end time of the period and/or the duration of the period.

The information for multiple sleep periods can be analysed. In some embodiments, the sleep periods can be evaluated to determine a measure of their regularity or variability. In some embodiments, a variability measure can be determined based on information for multiple sleep periods across different days. If the variation in the duration and/or start time of the sleeping/snoozing periods across several days or sleeping periods is more than a threshold value, then an indication, notification or message can be provided to a user of the apparatus 2, for example to the subject 14, care provider or other interested party, indicating that an intervention with the subject 14 may be required. A suitable value for the threshold value may be determined based on a population of subjects 14, or the threshold value may be set by a care provider, or other party. In an example, the information for the sleeping periods is arranged in a time series, and the variability is evaluated daily. Parameters that can be evaluated for variability include the total duration of sleep (in the day) per day, the duration of the main cluster of sleeping periods and/or largest sleeping period, the time of day of the largest (i.e. main cluster of) sleeping period, etc.). Trend analysis and change point detection can be applied to identify aberrations and alert for possible intervention with the subject 14. In change point detection, given a sequence of data points x[-n..k..m], it is tested whether the subsets x[-n..k-1] and x[k..m] have different parameters (like mean, variance, etc.). If so, the k sample is a change point. Similar approaches apply for trend analysis. It is also possible to observe values, as they occur over time, and test whether the current sample exceeds a threshold, for example to test if a sample is more than (e.g.) twice the standard deviation away from the mean.

The intervention with the subject 14 may comprise initiating a discussion between a care provider and the subject 14, including discussing the subject's night time sleeping routine. This intervention can be used by the care provider to ascertain whether there is a reason for the variation and whether any corrective action is required.

In embodiments where the sensor measurements are processed in real time or near-real time, if it is or has been determined that the variability or regularity in the daytime sleep periods is above the threshold value and the subject 14 is detected to be sleeping at an inappropriate time, then a stimulus could be provided to the subject 14 to wake the subject 14 up. This stimulus could be an acoustic stimulus (e.g. similar to an alarm clock), a tactile stimulus (e.g. a vibration) and/or a visual stimulus (e.g. a light). This stimulus could be provided by the user interface 8.

There is therefore provided a method and apparatus for monitoring a subject to determine when they have fallen asleep during the day.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus (2) for monitoring a subject (14), the apparatus (2) comprising a processing unit (4) that is configured to:
obtain measurements of a level of relaxation of a neck muscle of the subject (14) over time; and
process the measurements to identify one or more periods of time in which the subject (14) has fallen asleep, wherein the one or more periods of time in which the subject (14) has fallen asleep correspond to periods of time in which the neck muscle is relaxed.

2. An apparatus (2) as claimed in claim 1, wherein the one or more sensors (12) comprise one or more of an electromyography (EMG) sensor, an accelerometer, a gyroscope, a strain gauge, a sensor that measures the indentation of a probe into the neck muscle, a sensor that measures vibration of the muscle from an external stimulus and a camera.

3. An apparatus (2) as claimed in claim 2, wherein the one or more sensors (12) are configured to be worn or carried by the subject (14).

4. An apparatus (2) as claimed in claim 1, 2 or 3, wherein the apparatus (2) is configured to be worn at or around the neck of the subject (14).

5. An apparatus (2) as claimed in any of claims 1-4, wherein the apparatus (2) comprises a band, cord or strap that is to be worn around the neck of the subject (14), and wherein the one or more sensors (12) are arranged in or on the band, cord or strap such that the one or more sensors (12) are positionable adjacent to the neck muscle of the subject (14).

6. An apparatus (2) as claimed in any of claims 1-5, wherein the processing unit (4) is further configured to process i) the measurements of the level of relaxation of the neck muscle of the subject (14) and ii) measurements of one or more further characteristics of the subject (14), to identify one or more periods of time in which the subject (14) has fallen asleep.

7. An apparatus (2) as claimed in claim 6, wherein the measurements of one or more further characteristics of the subject (14) comprise one or more of a movement or activity level of the subject (14), a physiological characteristic of the subject (14), heart rate, heart rate variability, breathing rate, and skin conductivity.

8. An apparatus (2) as claimed in any of claims 1-7, wherein the processing unit (4) is further configured to:
determine a measure of the regularity or variability of a plurality of identified periods of time in which the subject (14) has fallen asleep.

9. An apparatus (2) as claimed in claim 8, wherein the processing unit (4) is further configured to:
compare the determined measure of the regularity or variability to a threshold value; and
provide an indication, notification or message to a user if the measure of the regularity or variability exceeds the threshold value.

10. A computer-implemented method of monitoring a subject, the method comprising:
obtaining (101) measurements of a level of relaxation of a neck muscle of the subject over time; and
processing (103) the measurements to identify one or more periods of time in which the subject has fallen asleep, wherein the one or more periods of time in which the subject has fallen asleep correspond to periods of time in which the neck muscle is relaxed.

11. A computer-implemented method as claimed in claim 10, wherein the step of processing (103) comprises processing i) the measurements of the level of relaxation of the neck muscle of the subject and ii) measurements of one or more further characteristics of the subject, to identify one or more periods of time in which the subject has fallen asleep.

12. A computer-implemented method as claimed in claim 11, wherein the measurements of one or more further characteristics of the subject comprise one or more of a movement or activity level of the subject, a physiological characteristic of the subject, heart rate, heart rate variability, breathing rate, and skin conductivity.

13. A computer-implemented method as claimed in any of claims 10-12, wherein the method further comprises:
determining a measure of the regularity or variability of a plurality of identified periods of time in which the subject has fallen asleep.

14. A computer-implemented method as claimed in claim 13, wherein the method further comprises:
comparing the determined measure of the regularity or variability to a threshold value; and
providing an indication, notification or message to a user if the measure of the regularity or variability exceeds the threshold value.

15. A computer program product comprising a computer readable medium having computer readable code embodied therein, the computer readable code being configured such that, on execution by a suitable computer or processor, the computer or processor is caused to perform the method of any claims 10-14.
